Europäisches Patentamt

European Patent Office

Office européen des brevets

(11) Veröffentlichungsnummer: **0 272 313 B1**

(19)

(12) **EUROPÄISCHE PATENTSCHRIFT**

(45) Veröffentlichungstag der Patentschrift: **31.10.90**

(21) Anmeldenummer: **87904521.9**

(22) Anmeldetag: **25.06.87**

(86) Internationale Anmeldenummer:
**PCT/EP87/00340**

(87) Internationale Veröffentlichungsnummer:
**WO 88/00069 14.01.88 Gazette 88/02**

(51) Int. Cl.⁵: **A 61 M 25/00, A 61 B 17/22**

(54) **SONDE ZUR EINFÜHRUNG IN DEN VERDAUUNGS- ODER AUSSCHEIDUNGSTRAKT DES MENSCHLICHEN ODER TIERISCHEN KÖRPERS, INSBESONDERE LITHOTRIPTOR-SONDE.**

(30) Priorität: **27.06.86 DE 3621558**

(43) Veröffentlichungstag der Anmeldung:
**29.06.88 Patentblatt 88/26**

(45) Bekanntmachung des Hinweises auf die Patenterteilung:
**31.10.90 Patentblatt 90/44**

(84) Benannte Vertragsstaaten:
**AT BE CH DE FR GB IT LI LU NL SE**

(56) Entgegenhaltungen:
**EP-A-0 131 847
EP-A-0 145 489
US-A-1 677 671
US-A-4 596 559**

(73) Patentinhaber: **Frimberger, Erintrud
Josef-Kösel-Weg 10
D-8960 Kempten (DE)**

(72) Erfinder: **FRIMBERGER, Eckart
Tristanstrasse 12
D-8000 München 40 (DE)**

(74) Vertreter: **Mitscherlich, Hans, Dipl.-Ing. et al
Patentanwälte Dipl.-Ing. H. Mitscherlich Dipl.-Ing. K. Gunschmann Dipl.-Ing. Dr.rer.nat. W. Körber Dipl.-Ing. J. Schmidt-Evers Dipl.-Ing. W. Melzer Steinsdorfstrasse 10
D-8000 München 22 (DE)**

Courier Press, Leamington Spa, England.

EP 0 272 313 B1

**Beschreibung**

Die Erfindung bezieht sich auf eine Sonde nach dem Oberbegriff des Anspruchs 1.

Eine Sonde dieser Art ist in der europäischen Patentanmeldung 0 131 847 beschrieben und dargestellt.

Steine im Gallengang können heute endoskopisch enfernt werden. Hierzu wird mit einem Endoskop, insbesondere einem flexiblen Seitblick Duodenoskop, die Mündungsstelle des Gallenganges im Zwölffingerdarm aufgesucht. In die Mündungsstelle (Papille) wird, ggf. nach vorausgegangener Schlitzung, die Sonde durch das Endoskop hindurch eingeführt. Um vorhandene Steine im Gallengang sichtbar zu machen, wird über die Sonde ein Röntgen-Kontrastmittel in den Gallengang eingespritzt. Der oder die Steine können nun mittels eines am freien Ende der Sonde angeordneten Korbes insbesondere aus Draht, durch die Mündungsstelle hindurchgezogen oder, falls die Steine zu groß sind, zerkleinert (lithotripsiert) werden.

Die eingangs bezeichnete bekannte Sonde umfaßt ein Führungsrohr, bestehend aus in seiner Längsrichtung aneinanderliegenden Wicklungen, einer im Führungsrohr verschiebbaren Seele, insbesondere aus Draht oder -litze, die mit dem Korb verbunden ist und mittels der der Korb zwischen einer in das Führungsrohr zurückgezogenen Position und einer aus dem Führungsrohr herausgeschobenen Position verschiebbar ist, und einer Spannvorrichtung bzw. einem Winder, der am äußeren Ende des Führungsrohrs abgestützt ist und mit einem Zugelement an das äußere Ende der Seele angreift.

Zum Zerkleinern eines Steines wird die Seele durcn den Winder zurückgezogen, wobei der Korb in die Sonde hereingezogen wird. Da der Stein größer ist als die Sonde ergibt sich eine auf den Stein gerichtete Spannung in den Drähten des Korbes, die in den meisten Fällen zur Zerkleinerung des Steines führt. Bei diesem Vorgang wird der den Stein umgreifende, sich vergrößernde Abschnitt des Korbes gegen das innere Ende der Sonde gepreßt, wodurch die vorgenannten Kräfte erzeugt werden, weil das Führungsrohr kompressionsstabil ist. Die Zerkleinerung kann ggf. solange fortgesetzt werden, bis die Steinteile eine deren Durchführung durch die Mündungsstelle nach außen gewährleistende Größe aufweisen.

Bei der vorbeschriebenen bekannten Ausgestaltung steht das bei der Manipulation ausgebogene Führungsrohr aufgrund der aneinanderliegenden Wicklungen unter einer beträchtlichen Spannung, die das Führungsrohr im Bereich seines aus dem Endoskop herausragenden Endes immer in seine gerade Erstreckung zurückzuführen sucht. Durch diese Spannung wird die Manipulation, hier die Führung des Korbes und Überführung auf einen Stein, ganz erheblich erschwert.

Es ist deshalb schon vorgeschlagen worden, das Führungsrohr aus einem Kunststoffrohr herzustellen, das aufgrund seiner Plastizität sich erheblich besser zur Führung des Korbes über einen Stein eignet. Das Kunststoffrohr ist jedoch mangels axialer Druckfestigkeit für die Ubertragung axialer Druckkräfte, hier für die Zerkleinerung eines Steins, nicht geeignet. Zur Ausübung einer axialen Druckkraft bedarf es deshalb eines Austausches des Kunststoffrohres gegen ein axial druckfestes Führungsrohr, was sehr umständlich und zeitraubend ist.

Der Erfindung liegt die Aufgabe zugrunde, eine Sonde der eingangs bezeichneten Art so auszugestalten, daß bei Gewährleistung der für das Zerkleinern des Steins erforderlichen Druckfestigkeit des Führungsrohrs die Manipulation mit dem Manipulationselement erleichtert ist.

Diese Aufgabe wird durch die kennzeichnenden Merkmale des Anspruchs 1 bzw. 7 gelöst.

Bei der erfindungsgemäßen Ausgestaltung gemäß Anspruch 1 weist das Führungsrohr an seinem inneren Ende ein plastisch verformbares Führungsstück auf, dessen Länge im wesentlichen der für die Manipulation erforderlichen Bewegungslänge entspricht. Aufgrund der plastischen Verform- bzw. Biegsamkeit des Führungsstücks kann es in für die Manipulation günstige Positionen gebogen werden, ohne daß die Gefahr besteht, daß es in seiner Ausgangsposition zurückschnellt. Dies ist insbesondere für eine Manipulation mittels einem Endoskop von Vorteil, weil die Ausrichtung des aus dem Endoskop herausragenden Endes des Führungsrohrs zum einen durch das Endoskop und zum anderen durch die Anatomie des zu behandelnden Körpers vorbestimmt ist. Bei der Manipulation kann die Steifigkeit des Führungsstückes, die es seiner Verformung entgegensetzt, zur Manipulation ausgenutzt werden, d.h., die Spannung, die das Führungsstück seiner Verbiegung entgegengesetzt, kann im Fall der Lithotripsie zur seitlichen Überführung des Korbes auf den Stein ausgenutzt werden. Bei der Ausübung einer Zugkraft auf die Seele der Sonde wird das Führungsstück soweit zusammengepreßt, bis seine Druckfestigkeit derart vergrößert ist, daß die angestrebte Zugkraft auf das Manipulationselement ausgeübt werden kann. Dabei kann sich das Führungsstück ziehharmonikaähnlich zusammendrücken. Der sich bei dieser Verformung ergebende Zugweg bleibt in vertretbaren Grenzen, weil aufgrund der verhältnismäßig kleinen Länge des Führungsstückes gegenüber dem Führungsrohr die erforderliche Druckfestigkeit bald erreicht wird.

Bei der erfindungsgemäßen Ausgestaltung nach Anspruch 7 weist ein Längsabschnitt des Führungsrohrs an seinem inneren Ende voneinander geringfügig beabstandete Wicklungen auf. Bei dieser Ausgestaltung tritt eine bedeutende Verbesserung gegenüber der bekannten Ausgestaltung deshalb ein, weil die schon als nachteilig beschriebene Spannung erheblich reduziert wird, und zwar dadurch, daß den Wicklungen die gegenseitige axiale Abstützung genommen wird und deshalb das Führungsrohr im Bereich des erfindungsgemäßen Längsabschnittes leichter verformt werden kann. Auch bei dieser Ausgestaltung ist eine plastische Verformbarkeit von Vorteil, wozu es einer Abstimmung des Materials der Wicklungen bedarf. Ein Material, das sich plastischverformen läßt, eignet sich deshalb besonders gut.

Bei beiden erfindungsgemäßen Ausgestaltungen ist die Länge des Führungsstückes nicht größer zu bemessen, als es für die jeweilige Manipulation erforderlich ist. Eine wesentlich größere Bemessung wird sich dann nachteilig auswirken, wenn das Führungsstück zwecks der Ausübung einer Zugkraft auf des Führungselement axial zusammengedrückt wird. Die Heranführung des Führungsstückes an den jeweiligen Manipulationsort kann z.B. dadurch herbeigeführt werden, daß das Führungsstück durch Verlagerung in die entgegengesetzte Richtung gegen eine Wand des zu behandelnden Körpers das Führungsstück soweit gebogen wird, daß es nach der Zurückführung sich im Bereich des Manipulationsortes befindet.

Die Ausgestaltung nach Anspruch 2 umfaßt eine einfache und funktionsfähige Anordnung und Formgebung für das Führungsstück, wobei die einfache Abstützung gegen das innere Ende des Führungsrohrs geeignet ist, die beim Einsatz auftretenden Zugkräfte zu übertragen.

Bei der Ausgestaltung nach Anspruch 5 ist das Führungsstück auch in der Zugrichtung der Seele entgegengesetzten Richtung mit dem Führungsrohr verbunden.

Von den in den Ansprüchen 3 und 4 angegebenen Ausgestaltungen bzw. Werkstoffen eignet sich ein Führungsrohr aus Kunststoff deshalb besonders gut, weil es eine glatte Oberfläche besitzt, die nicht nur eine leichte Verschiebung, sondern auch eine gründliche Reinigung bzw. Desinfektion ermöglicht.

Es ist von Vorteil, die Wicklungen in dem aufgepreßten Bereich des Führungsrohrs gemäß Anspruch 6 starr miteinander zu verbinden, insbesondere zu verschweißen, zu verlöten oder zu verkleben. Hierdurch wird der Sitz des muffenförmig auf das Führungsrohr aufgeschobenen Rohrstücks stabilisiert.

Durch eine leicht gebogene Ausführung des Rohrstücks oder Abschnitts gemäß Anspruch 8 wird die Manipulation, insbesondere das Erfassen bzw. Einfangen des Steins mit dem Korb, erleichtert. Die Ausrichtung der Krümmung kann an die sich jeweils ergebenden Erfordernisse dadurch angepaßt werden, daß die Sonde beispielsweise im Endoskop, wenn die Sonde durch ein solches in den Körper eingeführt wird, gedreht wird.

Bei Versuchen hat sich gemäß Anspruch 9 eine Länge des Rohrstücks bzw. Abschnitts von etwa 5 bis 15 cm als besonders vorteilhaft erwiesen.

Wie schon erwähnt worden ist, zieht sich bei der erfindungsgemäßen Ausgestaltung das aus einem Rohrstück gebildete Führungsstück ziehharmonikaähnlich zusammen, wenn eine Zugkraft auf das Manipulationselement ausgeübt wird. Die dabei zu verrichtende Arbeit kann entfallen, wenn das Rohrstück gemäß Anspruch 10 eine längsgerichtete Soll-Reißlinie aufweist, die sich bis nahe seinem äußeren Ende erstreckt. Bei einer solchen Ausbildung platzt das Rohrstück auf, so daß das Manipulationselement mit einem geringen Widerstand bis nahe vor das innere Ende des Führungsrohrs gezogen werden kann, wo es den erwünschten Anschlag findet. Da die Soll-Reißlinie sich nicht über die gesamte Länge des Rohrstückes erstreckt, bleibt letzteres an der Seele bzw. am Führungsrohr gehalten, so daß es nach Beendigung der Manipulation in das Endoskop eingezogen oder mit dem Endoskop aus dem Körper entfernt werden kann. Auf jeden Fall ist verhindert, daß das Rohrstück völlig getrennt wird und im Körper verbleibt. Bei der Ausgestaltung nach Anspruch 11 wird das Führungsstück aus den vorgenannten Gründen völlig von der Sonde getrennt. Es ist deshalb erforderlich, das Führungsstück nach der Manipulation aus dem Körper zu entfernen, was durch geeignete bekannte Mittel möglich ist.

Nachfolgend wird die Erfindung anhand von in vereinfachten Zeichnungen dargestellten bevorzugten Ausführungsbeispielen näher erläutert. Es zeigt

Fig. 1 eine erfindungsgemäß ausgestaltete Sonde in der Seitenansicht;

Fig. 2 das Einführungsende der Sonde mit einem aus der Sonde herausgeschobenen Korb;

Fig. 3 die in Fig. 1 mit X gekennzeichnete Einzelheit in vergrößerter Schnittdarstellung;

Fig. 4 eine Funktionsdarstellung mit der eingangs bezeichneten bekannten Ausgestaltung der Sonde;

Fig. 5 eine Funktionsdarstellung mit der erfindungsgemäßen Ausgestaltung der Sonde;

Fig. 6 ein zweites Ausführungsbeispiel der Sonde.

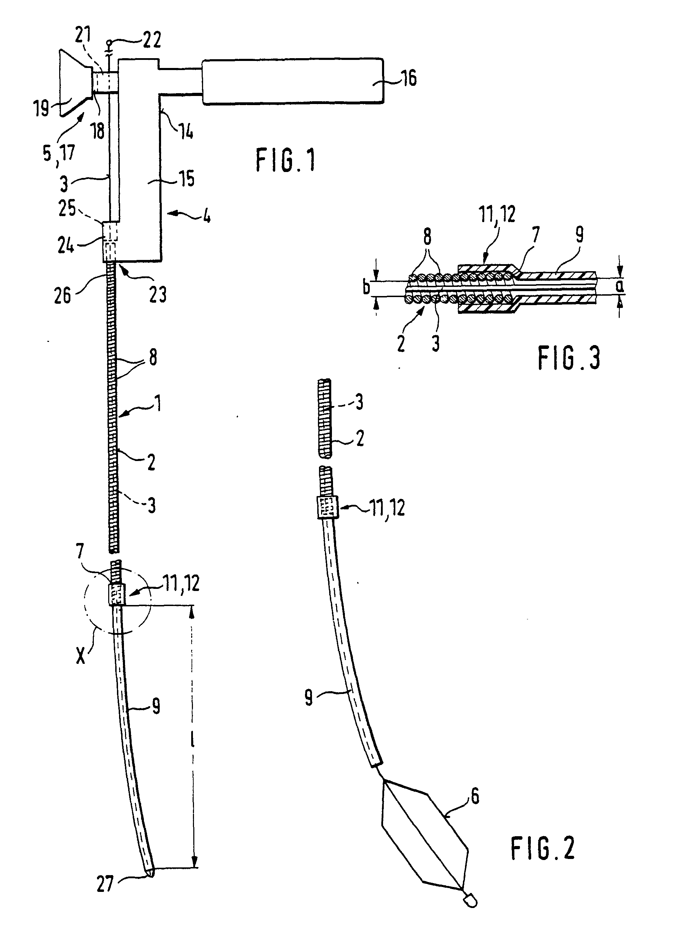

Die in Fig. 1 allgemein mit 1 bezeichnete Sonde besteht, wie bekannt, aus einem Führungsrohr 2, einer darin längs verschiebbaren Seele 3, einem sog. Winder 4, an dem das äußere Ende des Führungsrohrs 2 abgestützt ist und der ein Spannelement 5 aufweist, das an der Seele 3 angreift, und einem Manipulationselement in Form eines Korbes 6 am inneren Ende der Seele 3.

Das Führungsrohr 2 besteht aus in seiner Längsrichtung aneinanderliegenden, gewindeförmigen Wicklungen 8 aus Metall. An seinem inneren Ende 7 weist das Führungsrohr 2 ein plastisch biegsames Rohrstück 9 aus Kunststoff auf, das bei 11 muffenförmig auf die Wicklungen 8 aufgepreßt ist. Der Bereich, in dem das Rohrstück 9 die Wicklungen 8 übergreift, ist mit 12 bezeichnet. Der Innendurchmesser a des Rohrstücks 9 ist dem Innendurchmesser b des aus Wicklungen 8 bestehenden Führungsrohrs 2 angepaßt bzw. geringfügig größer bemessen als letzterer, so daß das Rohrstück 9 aufgrund seiner Elastizität mit einer Preßspannung auf den Wicklungen 8 sitzt. Die Führung der Seele 3 im Führungsrohr 2 wird durch die Befestigungsstelle 11 nicht beeinträchtigt.

Aufgrund der Preßspannung drücken sich die Wicklungen 8 in die Innenwandung des sie überdeckenden Bereichs 12 ein. Hierdurch erhält die Verbindung eine beachtliche Festigkeit. Zur weiteren Verstärkung dieser Festigkeit sind die Wicklungen 8 im Bereich 12 der Befestigungsstelle 11 miteinander verbunden, z.B. durch Schweißen, Löten oder Kleben.

Das Rohrstück 9 ist leicht gekrümmt was aus den Fig. 1, 2 und 5 deutlich zu entnehmen ist. Auf den hierdurch erzielbaren Vorteil wird weiter unten noch eingegangen.

Der Winder 4 besteht aus einem Handstück 14 mit einem Schaft 15 und einem von diesen rechtwinklig ausgehenden Griff 16, wobei auf der dem Griff 16 gegenüberliegenden Seite des Schaftes 15 ein Drehelement 17 in Form eines axial geschlitzten Zylinders 18 mit einem Drehkopf 19 rechtwinklig zum Schaft 15 in letzterem drehbar gelagert ist. Die Seele 3 erstreckt sich durch den Schlitz 21 im Zylinder 18 und kann vorzugsweise an ihrem äußeren Ende einen Kopf 22 tragen, der größer bemessen ist, als die Breite des Schlitzes 21, so daß der Kopf 22 nicht hindurchzurutschen vermag. Koaxial zu der Stelle, an der die Seele 3 den Schlitz 21 bzw. den Zylinder 18 durchsetzt, befindet sich am dem Drehelement 17 abgewandten Ende des Schaftes 15 eine Einsecköffnung 23 für das Führungsrohr 2 in einem seitlichen Ansatz 24, die zur dem Drehelement 17 abgewandten Seite des Schaftes 15 offen und koaxial zu einem Schlitz 25 im Ansatz 24 für die Seele 3 angeordnet ist. Zum Festlegen des äußeren Endes 26 des Führungsrohrs 2 wird zunächst die Seele 3 in den Schlitz 25 eingeführt und dann das äußere Ende 26 in die Einsecköffnung 23 eingesteckt. Durch Drehen des Drehelementes 17 erfolgt dann eine Aufwicklung der Seele 3 auf den Zylinder 18, wodurch eine Zugkraft bzw. eine rückwärtige Bewegung auf die Seele 3 übertragen werden kann.

Bei der in Fig. 1 dargestellten Ausgangsposition ist der Korb 6 in das Führungsrohr 2 bzw. in das Rohrstück 9 zurückgezogen und zwar bis zu einem Anschlagkopf 27 an seinem freien Ende.

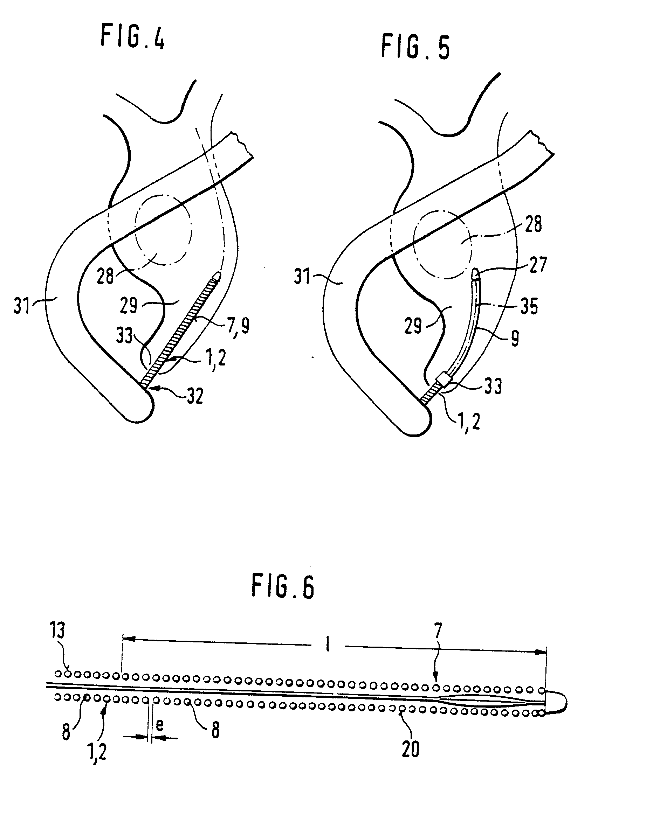

Beim Einsatz der Sonde 1 zum Zerkleinern eines Steines 28 in einem Gallengang 29 (Fig. 4 und 5) wird die Sonde mittels eines flexiblen Endoskops 31, insbesondere eines Duodenoskops mit einem seitlichen Ausgang 32 für die Sonde 1 in die Mündungsstelle (Papille) des Gallengangs 29 eingeführt. In Fig. 4 ist die bekannte Ausgestaltung des Führungsrohrs 2 dargestellt, dessen inneres Ende 7 aufgrund der vorhandenen Spannung und der Anlage der Wicklungen 8 aneinander sich gerade erstreckt, wobei die Spannung das innere Ende 7 des etwa rechtwinklig aus dem Endoskop abstehenden Führungsrohrs 2 in der geraden Erstreckung zu halten sucht. Bei einer solchen Sonde ist es sehr schwierig, den in Fig. 2 in ausgefahrener Position dargestellten Korb 6 über den Stein 28 zu ziehen und diesen somit einzufangen. Dies wird dadurch verdeutlicht, daß das innere Ende 7 des Führungsrohrs 2 während seines Vorschiebens in den Gallengang 29 den mit einer Strichpunktlinie dargestellten Weg einschlägt. Aus dieser Position ist der Stein 28 äußerst schlecht zu erfassen. Auch durch entsprechende Bewegungen des inneren Endes des Endoskops 31 erweist es sich als äußerst schwierig, den Stein 28 zu erfassen.

Bei der erfindungsgemäßen Ausgestaltung (Fig. 5) läßt sich das Rohrstück 9 dagegen leicht, z.B durch seitlichen Druck gegen die hier mit 33 bezeichneten Mündungsstelle oder die benachbarte Wand des Körpers in die Nähe des Steins 28 führen, wobei es in dieser Position verbleibt. Der Korb 6 kann den Stein 28 dann leicht erfassen.

Das Erfassen des Steins 28 ist insbesondere dann sehr erleichtert, wenn das Rohrstück 9 leicht gekrümmt ist, wie es in Fig. 5 dargestellt ist, in der es von vorneherein eine auf den Stein 28 gerichtete günstige Ausgangsposition hat. Die Ausrichtung der Krümmung kann durch ein Drehen der Sonde 1 im Endoskop, wenn sie in einem solchen eingeführt worden ist, an die Erfordernisse angepaßt werden.

Zum Zerkleinern des größer als die Mündungsstelle 33 vorliegenden Steins 28 wird der Korb 6 durch Drehen des Drehelementes 17 in die entsprechende Richtung zurückgezogen, wobei das Rohrstück 9 axial komprimiert wird, bis sich ein solcher Kompressionswiderstand einstellt, der an den Drähten des Korbes 6 für das Spalten des Steins 28 erforderliche Kräfte erzeugt. Das Rohrstück 9 kann dabei ziehharmonikaähnlich zusammengeschoben werden.

Eine solche Deformierung des Rohrstückes 9 kann durch ein teilweises Ablösen des Rohrstücks 9 von der Seele 3 vermieden werden. Hierzu ist eine andeutungsweise durch eine Strichpunkt-Linie dargestellte, sich längs erstreckende Soll-Reißlinie 35 am Rohrstück 9 vorgesehen, die in der Nähe des äußeren Endes des Rohrstückes 9 endet. Auf diese Weise erfolgt keine vollständige Ablösung des Rohrstückes 9 von der Seele 3, da das äußere Ende des Rohrstückes 9 an der Seele 3 hängen bleibt.

Eine vorbeschriebene Deformierung aufgrund der Kompression oder des Trennens kann den Rückzug des Rohrstückes 9 durch das Endoskop 31 aufgrund von Formvergrößerung gefährden. Diese Schwierigkeit läßt sich dadurch beseitigen, daß die Soll-Reißlinie 35 auf der ganzen Länge des Rohrstückes 9 angeordnet wird. Bei einer solchen Ausgestaltung erfolgt unter der Kompression eine vollständige Ablösung des Rohrstücks 9, so daß die Sonde 1 problemlos zurückgezogen werden kann. Das im Körper verbleibende, abgelöste Rohrstück 9 kann anschließend durch eine besondere Sonde entfernt werden. In dem Fall, in dem das Rohrstück 9 teilweise von der Seele 2 abgelöst noch an der Seele 3 hängt, wird es beim Herausziehen des Endoskops 31 aus dem Körper entfernt. Die Soll-Reißlinie 35 kann durch eine linienförmige Schwächung, einen Anschnitt der Wandung oder durch eine Perforation gebildet sein.

Beim zweiten Ausführungsbeispiel gemäß Fig. 6 weist das Führungsrohr 2 an einem inneren Ende 7 einen Längsabschnitt 20 auf, in dessen Bereich die Wicklungen 8 geringfügig voneinander beabstandet sind (Abstand e). Hierdurch kann die eingangs bezeichnete unerwünschte Spannung des Führungsrohrs 2 erheblich verringert oder auch aufgehoben werden, wodurch der Längsabschnitt 20 leichter geführt bzw. manipuliert werden kann. Die Führung des inneren Endes der Seele 3 einschließlich des Korbes 6 bleibt dabei aufrecht erhalten.

## Patentansprüche

1. Sonde zur Einführung in den Verdauungs- oder Ausscheidungtrakt des menschlichen oder tierischen Körpers, insbesondere Lithotriptor-Sonde,

mit einem biegeelastischen, in seiner Längsrichtung druckfesten Führungsrohr (2),

einer darin verschiebbaren Seele (3), insbesondere aus Draht oder -litze,

einem am äußeren Ende (26) des Führungsrohrs (2) abgestützten Spannteil (4), das mit seinem Spannelement (5) an der Seele (3) angreift,

und einem Manipulationselement am inneren Ende der Seele (3), das beim Manipulieren vor dem inneren Ende (7) des Führungsrohrs (2) angeordnet ist und durch eine Zugbewegung gegen das innere Ende (7) des Führungsrohrs (2) in Funktion setzbar ist, insbesondere einem Korb (6) dadurch gekennzeichnet,

daß sich das Führungsrohr (2) in einem an seinem inneren Ende (7) angeordnetes, plastisch biegsames Führungsstück (9) für die Seele (3), fortsetzt, dessen Länge (1) im wesentlichen der für die Manipulation erforderlichen Bewegungslänge entspricht.

2. Sonde nach Anspruch 1, dadurch gekennzeichnet, daß das Führungsstück ein Rohrstück (9) ist, das stirnseitig am inneren Ende des Führungsrohrs (2) anliegt.

3. Sonde nach Anspruch 1 oder 2, dadurch gekennzeichnet, daß das Führungsrohr (2) aus in seiner Längsrichtung aneinanderliegenden Wicklungen (8) besteht.

4. Sonde nach Anspruch 1 oder 2, dadurch gekennzeichnet, daß das Führungsrohr (2) eine durchgehende Wandung aus Kunststoff aufweist.

5. Sonde nach Anspruch 2 und 3, dadurch gekennzeichnet, daß das Rohrstück (9) muffenförmig, vorzugsweise unter Aufweitung seines Querschnitts auf den Wicklungen (8) des Führungsrohrs (3) sitzt.

6. Sonde nach Anspruch 5, dadurch gekennzeichnet, daß in dem aufgesetzten Bereich (12) die Wicklungen (8) miteinander verbunden sind, vorzugsweise durch Schweißen, Löten oder Kleben.

7. Sonde zur Einführung in den Verdauungsoder Ausscheidungsttrakt des menschlichen oder tierischen Körpers, insbesondere Lithotriptor-Sonde,

mit einem biegeelastischen Führungsrohr (2), bestehend aus in seiner Längsrichtung aneinanderliegendenden Wicklungen (8),

einer darin verschiebbaren Seele (3), insbesondere aus Draht oder -litze, einem am äußeren Ende (26) des Führungsrohrs (2) abgestützten Spannteil (4), das mit einem Spannelement (5) an der Seele (3) angreift,

und einem Manipulationselement am inneren Ende der Seele (3), das beim Manipulieren vor dem inneren Ende (7) des Führungsrohrs (2) angeordnet ist und durch eine Zugbewegung gegen das innere Ende (7) des Führungsrohrs (2) in Funktion setzbar ist, insbesondere einem Korb (6), dadurch gekennzeichnet,

daß sich das Führungsrohr (2) in einem au seinem inneren Ende (7) au-geordneten Längsabschnitt (20) mit voneinander beabstandeten (Abstand e) Wicklungen (8) fortsetzt (Fig. 6).

8. Sonde nach einem der Ansprüche 1 bis 7, dadurch gekennzeichnet, daß das Führungsstück (9) oder der Längsabschnitt (20) leicht gekrümmt ist.

9. Sonde nach einem der Ansprüche 1 bis 8, dadurch gekennzeichnet, daß die Länge (1) des Führungsstücks (9) oder des Längsabschnitts (20) etwa 5 bis 15 cm beträgt.

10. Sonde nach einem der Ansprüche 1 bis 6 und 8 sowie 9, dadurch gekennzeichnet, daß das Rohrstück (9) eine längs gerichtete Soll-Reißlinie (35) aufweist, die nahe seinem äußeren Ende endet.

11. Sonde nach einem der Ansprüche 1 bis 6 und 8 sowie 9, dadurch gekennzeichnet, daß das Rohrstück (9) eine längs durchgehende SollReißlinie aufweist.

12. Sonde nach einem der Ansprüche 1 bis 11, dadurch gekennzeichnet, daß das Führungsstück (9) aus Kunststoff besteht.

## Revendications

1. Sonde destinée à être introduite dans le tractus excréteur ou éliminateur du corps humain ou animal, en particulier sonde lithotriteur,

comprenant un tube de guidage (2) élastique en flexion, rigide à la compression dans sa direction longitudinale, une âme (3) pouvant coulisser dans ce tube, notamment composée d'un fil ou toron métallique,

une partie de serrage (4) qui prend appui à l'extrémité extérieure (26) du tube de guidage (2) et qui attaque l'âme (3) à l'aide de son élément de serrage (5),

et un élément manipulateur, en particulier une cage, prévu à l'extrémité intérieure de l'âme (3), qui est disposé, lors de la manipulation, en avant de l'extrémité intérieure (7) du tube de guidage (2) et qui peut être mis en action par un mouvement de traction vers l'extrémité intérieure (7) du tube de guidage (2),

caractérisée en ce que le tube de guidage (2) se prolonge par une pièce de guidage (9) servant à guider l'âme (3), disposée à son extrémité intérieure (7) et pouvant subir une déformation plastique par flexion, et dont la longueur (1) correspond sensiblement à la longueur de déplacement nécessaire pour la manipulation.

2. Sonde selon la revendication 1, caractérisée en ce que la pièce de guidage est un tronçon de tube (9) qui s'appuie, à une extrémité frontale, contre l'extrémité intérieure du tube de guidage (2).

3. Sonde selon la revendication 1 ou 2, caractérisée en ce que le tube de guidage (2) est constitué par des spires (8) qui sont mutuellement adjacentes dans la direction longitudinale de ce tube.

4. Sonde selon la revendication 1 ou 2, caractérisée en ce que le tube de guidage (2) présente une paroi continue en matière plastique.

5. Sonde selon l'une des revendications 2 et 3, caractérisée en ce que le tronçon de tube (9) est emboîté à la façon d'un manchon sur les spires (8) du tube de guidage (3), de préférence avec élargissement de sa section.

6. Sonde selon la revendication 5, caractérisée en ce que, dans la région emboîtée (12), les spires (8) sont assemblées l'une à l'autre, de préférence par soudage, brasage ou collage.

7. Sonde destinée à être introduite dans le tractus excréteur ou éliminateur du corps humain ou animal, notamment sonde lithotriteur, comprenant un tube de guidage (2) élastique en flexion, composé de spires (8) appuyées les unes contre les autres dans sa direction longitudinale, une âme (3) pouvant coulisser dans ce tube, notamment composée d'un fil ou toron métallique, une partie de serrage (4) qui prend appui à l'extrémité extérieure (26) du tube de guidage (2), et qui attaque l'âme (3) à l'aide de son élément de serrage (5), et un élément manipulateur, en particulier une cage (6), prévu à l'extrémité intérieure de l'âme (3), qui est disposé en avant de l'extrémité intérieure (7) du tube de guidage (2) lors de la manipulation, et peut être mis en action par un mouvement de traction dirigé vers l'extrémité intérieure (7) du tube de guidage (2), caractérisée en ce que le tube de guidage (2) se prolonge par un segment longitudinal (20) agencé à son extrémité intérieure (7), et qui comprend des spires (8) espacées les unes des autres (écartement e) (figure 6).

8. Sonde selon l'une des revendications 1 à 7, caractérisée en ce que la pièce de guidage (9) ou le segment longitudinal (20) est légèrement recourbé.

9. Sonde selon l'une des revendications 1 à 8, caractérisée en ce que la longueur (1) de la pièce de guidage (9) ou du segment longitudinal (20) est d'environ 5 à 15 cm.

10. Sonde selon l'une des revendications 1 à 6 et 8 ainsi que 9, caractérisée en ce que le tronçon de tube (9) possède une ligne d'amorce de déchirure (35) dirigée dans la direction longitudinale qui se termine à proximité de son extrémité extérieure.

11. Sonde selon l'une des revendications 1 à 6 et 8 ainsi que 9, caractérisée en ce que le tronçon de tube (9) présente une ligne d'amorce de déchirure continue dans la direction longitudinale.

12. Sonde selon l'une des revendications 1 à 11, caractérisée en ce que la pièce de guidage (9) est composée de matière plastique.

## Claims

1. A probe for introduction into the digestive or excretory tract of the human or animal body, in particular a lithotriptor probe, having an elastically flexible guide tube (2) that can withstand pressure in the longitudinal direction, a displaceable core (3), in particular of wire or stranded wire, therein, a tension part (4) supported at the outer end (26) of the guide tube (2) with a tension element (5) that engages with the core (3), and a manipulation element, in particular a basket (6), at the inner end of the core (3) that during the manipulation is arranged in front of the inner end (7) of the guide tube (2) and can be set into operation against the inner end (7) of the guide tube (2) by a tractive movement, characterised in that the guide tube (2) extends in a plastically flexible guide piece (9) for the core (3) arranged at its inner end (7), the length (1) of which substantially corresponds to the extent of movement required for the manipulation.

2. A probe according to claim 1, characterised in that the guide piece is a section of tube (9) abutting at one end against the inner end of the guide tube (2).

3. A probe according to claim 1 or claim 2, characterised in that the guide tube (2) consists of windings (8) that adjoin one another along its length.

4. A probe according to claim 1 or claim 2, characterised in that the guide tube (2) has a continuous wall of plastics material.

5. A probe according to claim 2 and claim 3, characterised in that the section of tube (9) fits in the form of a sleeve, preferably with a widened cross-section, over the windings (8) of the guide tube (3).

6. A probe according to claim 5, characterised in that, in the region (12) in which the sleeve is fitted on, the windings (8) are joined together, preferably by welding, brazing or adhesion.

7. A probe for introduction into the digestive or excretory tract of the human or animal body, in particular a lithotriptor probe, having an elastically flexible guide tube (2) consisting of windings (8) that adjoin one another along its length, a displaceable core (3), in particular of wire or stranded wire, therein, a tension part (4) supported at the outer end (26) of the guide tube (2) with a tension element (5) that engages with the core (3) and a manipulation element, in particular a basket (6), at the inner end of the core (3), that during the manipulation is arranged in front of the inner end (7) of the guide tube (2) and can be set into operation against the inner end (7) of the guide tube (2) by a tractive movement, characterised in that the guide tube (2) continues in a portion (20) of its length arranged at its inner end (7) with windings (8) spaced apart from one another (spacing e) (Fig. 6).

8. A probe according to any one of claims 1 to 7, characterised in that the guide piece (9) or the portion (20) of its length is slightly curved.

9. A probe according to any one of claims 1 to 8, characterised in that the length (1) of the guide piece (9) or of the portion (20) of its length amounts to about 5 to 15 cm.

10. A probe according to any one of claims 1 to 6 and 8 and 9, characterised in that the section of tube (9) has a longitudinal tearing line (35) ending near its outer end.

11. A probe according to any one of claims 1 to 6 and 8 and 9, characterised in that the section of tube (9) has a continuous longitudinal tearing line.

12. A probe according to any one of claims 1 to 11, characterised in that the guide piece (9) consists of plastics material.

FIG.1

FIG.3

FIG.2

## FIG.4

## FIG.5

## FIG.6